# EUROPÄISCHE PATENTSCHRIFT

(11) **EP 3 997 442 B1**
(45) Veröffentlichungstag und Bekanntmachung des Hinweises auf die Patenterteilung: **11.03.2026**
(21) Anmeldenummer: 20746111.2
(22) Anmeldetag: 09.07.2020
(51) Int. Cl.: G01N 15/14, G01N 33/49, A61B 8/06, A61M 1/36, G01N 29/14, A61B 5/1455, A61B 5/026

(54) **BLUTUNTERSUCHUNGSGERÄT**
BLOOD TESTING DEVICE
APPAREIL D'ANALYSE DU SANG

(30) Priorität: 09.07.2019 LU 101301
(43) Veröffentlichungstag der Anmeldung: 18.05.2022
(73) Patentinhaber: Hemovent GmbH, 52076 Aachen (DE)
(72) Erfinder: MARSEILLE, Oliver, 52066 Aachen (DE); FRANZEN, Christopher, 52066 Aachen (DE)
(74) Vertreter: Sattler de Sousa e Brito, Clara
(86) Internationale Anmeldenummer: PCT/EP2020/069377
(87) Internationale Veröffentlichungsnummer: WO 2021/005159

(56) Entgegenhaltungen:
- WO-A1-00/47248
- WO-A1-2017/068081
- DE-A1- 102013 014 097

## Beschreibung

Die Erfindung betrifft ein Blutuntersuchungsgerät zum unmittelbaren Ankoppeln an eine Durchflussvorrichtung für Blut, insbesondere eine Durchflussvorrichtung eines extrakorporalen Blutkreislaufs, und zum Erfassen von Blutparametern des durch die Durchflussvorrichtung strömenden Blutes.

Die Erfindung betrifft außerdem eine Durchflussvorrichtung, die dazu ausgebildet und bestimmt ist, in einen extrakorporalen Blutkreislauf derart eingefügt zu werden, dass Blut entlang einer Durchflussrichtung durch die Durchflussvorrichtung strömt und die zur unmittelbaren Ankopplung an ein erfindungsgemäßes Blutuntersuchungsgerät ausgebildet ist.

Die Erfindung betrifft außerdem ein System, das ein solches Blutuntersuchungsgerät sowie eine solche Durchflussvorrichtung aufweist.

Insbesondere Blutuntersuchungsgeräte sind aus dem Stand der Technik bekannt.

Ferner ist aus der WO 00/ 47248 A1 eine mobile Herz-Lungen-Maschine mit im Wesentlichen zwei Modulen bekannt, welche zu einer funktionalen Einheit zusammenfügbar sind, nämlich einem blutführenden Einwegmodul, welches unter anderem Sensoren zur Blutuntersuchung aufweist, und einem nicht blutführenden wiederverwendbaren Modul, welches unter anderem einen Akkumulator zur Energieversorgung und auch einen Pumpenantrieb aufweist.

Aus der DE 10 2013 014 097 A1 ist ein Einwegartikel zur Verwendung in einer Dialysebehandlung bekannt, wobei der Einwegartikel wenigstens einen Sensor zur Ermittlung wenigstens eines die Dialyse betreffenden Messwertes aufweist.

Darüber hinaus ist aus der WO 201 7/ 068 081 A1 ein Zwischenelement für eine medizintechnische extrakorporale Fluidleitung bekannt, welche dazu eingerichtet ist, ein Fluid, wie Blut, zu führen. Das Zwischenelement weist einen Basiskörper auf, der sich zwischen zwei Verbindungsteile erstreckt und mit der Fluidleitung hydraulisch verbindbar ist. Der Basiskörper weist hierbei eine Aufnahme auf, welche dazu eingerichtet ist, einen Messwertgeber aufzunehmen. An der Aufnahme im Basiskörper ist ein Durchbruch angeordnet, der über ein elastisches Element zur Aufnahme hin fluiddicht ist. Ferner ist die Aufnahme dazu eingerichtet, einen Gassensor einer Sensoreinrichtung zur Messung wenigstens eines in dem Fluid enthaltenen Gases aufzunehmen. Das elastische Element ist ein Diffusionselement, welches permeabel für wenigstens ein Gas ist. Das Diffusionselement ist in einem Verbindungsbereich stoffschlüssig mit einem Rand des Durchbruchs verbunden.

Es ist die Aufgabe der vorliegenden Erfindung ein verbessertes Blutuntersuchungsgerät anzugeben.

Die Aufgabe wird durch ein Blutuntersuchungsgerät zum Erfassen von Blutparametern von Blut in einem extrakorporalen Blutkreislauf außerhalb einer Herz-Lungen-Maschine gelöst, wobei das Blutuntersuchungsgerät eine Aufnahme zum unmittelbaren Ankoppeln an eine Durchflussvorrichtung für Blut aufweist, welche mit dem extrakorporalen Blutkreislauf verbindbar ist, wobei das Blutuntersuchungsgerät kabellos betreibbar ist und hinsichtlich seiner Energieversorgung gegenüber anderen Geräten einer Herz-Lungen-Maschine autark ausgebildet ist, und wobei das Blutuntersuchungsgerät einen Meßgrößenaufnehmer und eine Anzeigevorrichtung zum Anzeigen von mittels des Meßgrößenaufnehmers erfassten und an die Anzeigevorrichtung weitergeleiteten Blutparametern aufweist.

Vorteilhaft ist es auch eine Durchflussvorrichtung anzugeben, die es ermöglicht, mittels eines anzukoppelnden Blutuntersuchungsgeräts, gleichzeitig auf unterschiedliche Weise unterschiedliche Blutparameter einfach und schnell bestimmen zu können.

Die Aufgabe wird insofern durch ein System gelöst aufweisend die Durchflussvorrichtung, die dazu ausgebildet und bestimmt ist, in einen extrakorporalen Blutkreislauf derart eingefügt zu werden, dass Blut entlang einer Durchflussrichtung durch die Durchflussvorrichtung strömt und die zur unmittelbaren Ankopplung an das Blutuntersuchungsgerät ausgebildet ist, wobei die Durchflussvorrichtung wenigstens ein Meßgrößenaufnehmer-Ankoppelelement und wenigstens einen Durchflussvorrichtungs-Meßgrößenaufnehmer umfasst, und aufweisend das vorliegende Blutuntersuchungsgerät.

Das erfindungsgemäße Blutuntersuchungsgerät ist autark ausgebildet. Ein ganz besonderer Vorteil des erfindungsgemäßen Blutuntersuchungsgeräts ist es, dass es schnell und ohne auf den Anschluss an ein Stromversorgungsnetz oder an andere Geräte angewiesen zu sein zum Einsatz gebracht werden kann. Das erfindungsgemäße Blutuntersuchungsgerät wird ohne Kabel betrieben, so dass einerseits eine besonders gute Handhabbarkeit erzielt ist und andererseits eine Behinderung des den Patienten behandelnden Personals durch herumhängende Kabel vermieden oder gar ein Abreißen der Kabel durch umhergehendes Personal verhindert ist. Es wird keine Ankopplung an andere Geräte zur Steuerung, Bediendung, Energieversorgung oder Darstellung der Daten durch Anzeigedisplay oder Alarme benötigt. Somit kann es überall dort zum Einsatz kommen, wo die Messung einer oder aller vorgesehenen Parameter in einem blutführenden Schlauch bestimmt werden sollen. Anwendungen im Rahmen von Herz-Lungen-Maschinen, ECMO und ECLS (künstlichen Lungen, Herz, bzw. Herzkreislaufunterstützung), Organperfusion und andere Organersatz bzw. Unterstützungssysteme sind mögliche Einsatzbereiche.

Das Gerät weist eine Anzeige zur Darstellung der gewünschten Messparameter und einen oder mehrere Bedienknöpfe auf, mit denen die Darstellung der Messparameter und Alarme ausgewählt oder geändert werden kann. Auch kann das Gerät über den Bedienknopf nur bei Bedarf aktiviert werden, um Energie zu sparen.

Außerdem ist das Blutuntersuchungsgerät, vorzugsweise als ein Handgerät, derart kompakt ausbildbar, dass es in einfacher Weise von einer Person transportiert und zum Einsatz gebracht werden kann. So wird das Gerät leichter als 1 kg, insbesondere leichter als 0,5 kg sein und ein Gehäuse kleiner als 10x10x10 cm insbesondere kleiner als 10x6x6 cm aufweisen. Das Gerät ist insbesondere so ausgeführt, dass es keine Halterung benötigt, sondern lediglich am blutführenden Schlauch fixiert wird.

Bei einer vorteilhaften Ausführungsform weist das Blutuntersuchungsgerät ein Gehäuse auf, in oder an dem ein elektrischer Energiespeicher und die Anzeigevorrichtung zum Anzeigen der erfassten Blutparameter angeordnet ist, bei der durch ein Kabel, beispielsweise ein USB Kabel, auch im Betrieb die interne Stromquelle geladen, unterstützt oder ersetzt werden kann. Die optionale Verbindung kann auch genutzt werden, um Daten zu übertragen oder auszulesen. Auch ein Laden mittels drahtloser Energieübertragung ist möglich.

Das erfindungsgemäße Blutuntersuchungsgerät weist eine Vorrichtung zur drahtlosen Datenübertragung, beispielsweise via Bluetooth oder einen RF-Link, für externe Monitore auf. Wenn ein Patient nach dem Transport in eine Klinikumgebung kommt, kann das Blutuntersuchungsgerät so direkt in das Klinikmonitoring eingekoppelt werden.

Bei einer besonderen Ausführungsform weist das Blutuntersuchungsgerät einen Meßgrößenaufnehmer auf. Insbesondere kann der Meßgrößenaufnehmer dazu ausgebildet und bestimmt sein, mit einem Meßgrößenaufnehmer-Ankoppelelement einer Durchflussvorrichtung in Wirkverbindung zu treten. Das Meßgrößenaufnehmer-Ankoppelelement kann beispielsweise ein Fenster oder eine Membran sein, was weiter unten noch im Detail erläutert ist. Grundsätzlich ist vorzugsweise vorgesehen, dass eine Wirkverbindung des Meßgrößenaufnehmers des Blutuntersuchungsgeräts mit einem Meßgrößenaufnehmer-Ankoppelelement einer Durchflussvorrichtung hergestellt wird, um Blutwerte zu messen. Um die Wirkverbindung herzustellen, werden vorzugsweise das Blutuntersuchungsgerät und die Durchflussvorrichtung aneinander, insbesondere formschlüssig, festgelegt. Außerdem erfolgt das Festlegen vorzugsweise derart, dass der Meßgrößenaufnehmer unmittelbar an dem Meßgrößenaufnehmer-Ankoppelelement anliegt oder mit ihm unmittelbar in Kontakt kommt.

Besonders vorteilhaft ist eine Ausführungsform, bei der der Meßgrößenaufnehmer wenigstens eine Lichtquelle zum Beaufschlagen des durch eine Durchflussvorrichtung fließenden Blutes mit Licht aufweist. Hierdurch kann ein bestimmter Lichteintrag in das Blut mittels des Meßgrößenaufnehmers erfolgen und von dem Blut, auf Grund der Beaufschlagung mit Licht, ausgehendes Detektionslicht (z.B. Reflexionslicht, Streulicht oder Fluoreszenzlicht) mittels eines Lichtsensors des Meßgrößenaufnehmers detektiert und gegebenenfalls, insbesondere hinsichtlich der Lichtleistung und/oder der Wellenlänge, analysiert werden.

Alternativ oder zusätzlich kann vorteilhaft vorgesehen sein, dass der Meßgrößenaufnehmer wenigstens eine Schallquelle zum Beaufschlagen des durch eine Durchflussvorrichtung fließenden Blutes mit Schall aufweist. Hierdurch kann ein bestimmter Schalleintrag in das Blut mittels des Meßgrößenaufnehmers erfolgen. Es kann außerdem ein Schallsensor vorhanden sein, der vom Blut reflektierten oder gestreuten Schall empfängt. Ein solcher Meßgrößenaufnehmer kann insbesondere ein Ultraschall-Meßgrößenaufnehmer sein, der auf der Grundlage von Ultraschall funktioniert und als Durchflussmesser fungiert.

Besonders vorteilhaft ist eine Ausführungsform bei der der Meßgrößenaufnehmer sowohl eine Lichtquelle, als auch eine Schallquelle aufweist. Dadurch können gleichzeitig sowohl Blutparameter, die mittels Meßgrößenaufnehmer mit einer Lichtquelle, als auch mit einer Schallquelle zu bestimmen sind, aufgenommen werden.

Eine einfache Handhabbarkeit ist bei einer Ausführungsform gegeben, bei der das erfindungsgemäße Blutuntersuchungsgerät eine Meßgrößenaufnehmerschnittstelle aufweist, die dazu ausgebildet und bestimmt ist, mit einem in eine Durchflussvorrichtung integrierten Durchflussvorrichtungs-Meßgrößenaufnehmer in Wirkverbindung zu treten. Hierdurch ist es ermöglicht, das Blutuntersuchungsgerät zusammen mit einer Durchflussvorrichtung zu verwenden, die einen integrierten Meßgrößenaufnehmer beinhaltet, wobei ein unkompliziertes und schnelles Ankoppeln, insbesondere ohne zusätzliche Kabelverbindungen, ermöglicht ist.

Insbesondere kann die Meßgrößenaufnehmerschnittstelle vorteilhaft dazu ausgebildet sein, Energie von einem elektrische Energiespeicher des Blutuntersuchungsgeräts zu einem in eine Durchflussvorrichtung integrierten Durchflussvorrichtungs-Meßgrößenaufnehmer zu übertragen und/oder dazu ausgebildet sein, Messsignale von einem in eine Durchflussvorrichtung integrierten Durchflussvorrichtungs-Meßgrößenaufnehmer weiter zu leiten. Auf diese Weise ist eine unmittelbare Inbetriebnahme des in die Durchflussvorrichtung integrierten Durchflussvorrichtungs-Meßgrößenaufnehmers unmittelbar durch Ankoppeln des erfindungsgemäßen Blutuntersuchungsgeräts ermöglicht, ohne dass zusätzliche Kabelverbindungen zur Energie- oder Signalübertragung angeschlossen werden müssen.

Der Meßgrößenaufnehmer kann insbesondere vorteilhaft einen Druckmeßgrößenaufnehmer oder einen Durchflussmeßgrößenaufnehmer oder einen optischen Meßgrößenaufnehmer aufweisen. Insbesondere ist es auch möglich, dass mehrere Meßgrößenaufnehmer, insbesondere unterschiedlicher Art, vorhanden sind. Auf diese Weise sind verschiedene technische Untersuchungsmöglichkeiten, insbesondere auch in Kombination, realisierbar.

Bei einer einfach handhabbaren Ausführungsform des Blutuntersuchungsgeräts ist eine Aufnahme vorhanden, in oder an der eine Durchflussvorrichtung, insbesondere zerstörungsfrei und/oder werkzeugfrei, festlegbar ist. Die Aufnahme ermöglicht ein schnelles und effizientes, insbesondere zerstörungsfreies, An- und Abkoppeln einer Durchflussvorrichtung. Außerdem ist durch die Aufnahme für einen Benutzer eine sofort erkennbare Festlegemöglichkeit geschaffen, wodurch das Risiko einer Fehlbedienung durch ein fehlerhaftes Ankoppeln reduziert werden kann. Insbesondere ist so gewährleistet, dass das Blutuntersuchungsgerät und die Durchflussrichtung korrekt und unversehrt miteinander gekoppelt werden können.

Die Aufnahme kann vorteilhaft derart ausgebildet sein, dass beim Einfügen einer Durchflussvorrichtung automatisch eine Wirkverbindung des Meßgrößenaufnehmers an das Meßgrößenaufnehmer-Ankoppelelement und/oder der Meßgrößenaufnehmerschnittstelle an den Durchflussvorrichtungs-Meßgrößenaufnehmer hergestellt wird. Hierdurch ergibt sich in vorteilhafter Weise eine effiziente Ankopplung der Durchflussvorrichtung an das Blutuntersuchungsgerät. Die Aufnahme kann alternativ oder zusätzlich derart ausgebildet sein, dass beim Einfügen einer Durchflussvorrichtung automatisch eine Wirkverbindung der Meßgrößenaufnehmerschnittstelle an den Durchflussvorrichtungs-Meßgrößenaufnehmer hergestellt wird, wodurch außerdem vorteilhaft Daten von einem Meßgrößenaufnehmer, der an oder in der Durchflussvorrichtung angeordnet ist, an das Blutuntersuchungsgerät übertragen werden können.

Bei einer besonderen Ausführungsform weist die Aufnahme wenigstens ein Befestigungselement zum Festlegen einer Durchflussvorrichtung auf. Hierdurch ist eine besonders zuverlässige Halterung der Durchflussvorrichtung an dem Blutuntersuchungsgerät gewährleistet. Alternativ kann die Durchflussvorrichtung ein Befestigungselement aufweisen, das die mechanische Verbindung zu der Aufnahme herstellt. Bei einer anderen Ausführung weist die Aufnahme ein Befestigungselement auf, das mit einem Gegenbefestigungselement der Durchflussvorrichtung zusammen wirkt, wodurch sich eine besonders stabile Befestigung ergibt.

Bei einer vorteilhaften Ausführungsform ist das Blutuntersuchungsgerät dazu ausgebildet wenigstens einen Blutparameter zu erfassen. Hierzu weist das Blutuntersuchungsgerät eine Signal- und Datenverarbeitungseinrichtung auf, die die von den Meßgrößenaufnehmer aufgenommenen Signale, wie zum Beispiel von Blutzellen reflektierter Schall, verarbeitet sowie entsprechende Blutparameter daraus bestimmt und zur Ausgabe an die Anzeigevorrichtung weiterleitet.

Insbesondere ist das Blutuntersuchungsgerät dazu ausgebildet, wenigstens einen der folgenden Blutparameter zu erfassen: Sauerstoffsättigung des Blutes, CO2 Gehalt des Blutes, Temperatur des Blutes, Druck des Blutes, Durchflussmenge des Blutes pro Zeit, Durchflussgeschwindigkeit des Blutes und Hämoglobinanteil im Blut. Dadurch können in vorteilhafter Weise viele Blutparameter, die für die Vitalfunktionen des Patienten relevant sind, insbesondere permanent, überwacht werden.

Die Durchflussvorrichtung ist dazu ausgebildet und bestimmt, in einen extrakorporalen Blutkreislauf derart eingefügt zu werden, dass Blut entlang einer Durchflussrichtung durch die Durchflussvorrichtung strömt und ist zur unmittelbaren Ankopplung an ein erfindungsgemäßes Blutuntersuchungsgerät geeignet. Die Durchflussvorrichtung weist wenigstens ein Meßgrößenaufnehmer-Ankoppelelement und wenigstens einen Durchflussvorrichtungs-Meßgrößenaufnehmer auf.

Mittels der Durchflussvorrichtung lassen sich somit zum einen ein Blutuntersuchungsgerät mechanisch besonders einfach verbinden und zum anderen besonders einfach eine Kopplung zur Übertragung von Energie und Daten, sowohl von der Durchflussvorrichtung zum Blutuntersuchungsgerät, als auch vom Blutuntersuchungsgerät zur Durchflussvorrichtung, herstellen.

Bei einer besonderen Ausführungsform weist der Durchflussvorrichtungs-Meßgrößenaufnehmer einen Druckmeßgrößenaufnehmer auf, um den Druck des Blutes in dem extrakorporalen Blutkreislauf zu erfassen.

Bei einer weiteren Ausführungsform weist der Durchflussvorrichtungs-Meßgrößenaufnehmer einen Durchflussmeßgrößenaufnehmer auf. Somit ist in vorteilhafter Weise erreicht, dass die Durchflussmenge des Blutes durch den extrakorporalen Blutkreislauf pro Zeiteinheit erfassbar ist.

Bei einer anderen Ausführungsform weist der Durchflussvorrichtungs-Meßgrößenaufnehmer einen optischen Meßgrößenaufnehmer auf. Eine solche Ausführung hat den Vorteil, dass beispielsweise die Sauerstoffsättigung, der CO2 oder der Hämoglobingehalt des Blutes bestimmt werden kann.

Die Durchflussvorrichtung kann vorteilhaft auch mehrere integrierte Meßgrößenaufnehmer, insbesondere mehrere der oben erwähnten Meßgrößenaufnehmer, aufweisen.

Alternativ oder zusätzlich kann das Meßgrößenaufnehmer-Ankoppelelement zur Ankopplung an einen Durchflussvorrichtungs-Meßgrößenaufnehmer des Blutuntersuchungsgeräts ausgebildet sein, wobei der Durchflussvorrichtungs-Meßgrößenaufnehmer einen Druckmeßgrößenaufnehmer oder einen Durchflussmeßgrößenaufnehmer oder einen optischen Meßgrößenaufnehmer aufweist. Dadurch können in vorteilhafter Weise verschiedene Meßgrößenaufnehmer eines Blutuntersuchungsgerätes in unterschiedlichen Kombinationen an die Durchflussvorrichtung angekoppelt werden.

Bei einer besonderen Ausführungsform kann der Durchflussvorrichtungs-Meßgrößenaufnehmer wenigstens eine Lichtquelle zum Beaufschlagen des durch die Durchflussvorrichtung fließenden Blutes mit Licht aufweisen. Hierdurch kann ein bestimmter Lichteintrag in das Blut mittels des Meßgrößenaufnehmers erfolgen und von dem Blut, auf Grund der Beaufschlagung mit Licht, ausgehendes Detektionslicht (z.B. Reflexionslicht, Streulicht oder Fluoreszenzlicht) mittels eines Lichtsensors des Meßgrößenaufnehmers detektiert und gegebenenfalls, insbesondere hinsichtlich der Lichtleistung und/oder der Wellenlänge, analysiert werden.

Alternativ oder zusätzlich kann vorteilhaft vorgesehen sein, dass der Durchflussvorrichtungs-Meßgrößenaufnehmer wenigstens eine Schallquelle zum Beaufschlagen des durch eine Durchflussvorrichtung fließenden Blutes mit Schall aufweist. Hierdurch kann ein, insbesondere einstellbarer, Schalleintrag in das Blut mittels des Meßgrößenaufnehmers erfolgen. Es kann außerdem ein Schallsensor vorhanden sein, der vom Blut reflektierten oder gestreuten Schall empfängt. Ein solcher Meßgrößenaufnehmer kann insbesondere ein Ultraschall-Meßgrößenaufnehmer sein, der auf der Grundlage von Ultraschall funktioniert und als Durchflussmesser fungiert.

Besonders vorteilhaft ist eine Ausführungsform, bei der der Meßgrößenaufnehmer sowohl eine Lichtquelle, als auch eine Schallquelle aufweist. Dadurch können gleichzeitig mehrere, insbesondere unterschiedliche, Blutparameter, erfasst werden.

Insbesondere kann vorteilhaft vorgesehen sein, dass das Meßgrößenaufnehmer-Ankoppelelement ein Fenster aufweist. Durch das Fenster ist ein Sichtkontakt, beispielsweise zeitlich vor dem Ankoppeln eines Blutuntersuchungsgerätes an die Durchflussvorrichtung, zu dem durch die Durchflussvorrichtung strömenden Blut ermöglicht. Es können zum Beispiel in vorteilhafter Weise Farbveränderungen im Blut, die auf eine Veränderung des Hämoglobingehaltes im Blut hinweisen, von medizinischem Personal unmittelbar gesehen werden.

Außerdem ist eine effiziente Ankopplung eines optischen Meßgrößenaufnehmers möglich, da von einer Lichtquelle des optischen Meßgrößenaufnehmers abgegebenes Licht und/oder von einem Sensor des optischen Meßgrößenaufnehmers zu empfangenes Licht durch das Fenster hindurch propagieren kann.

Alternativ oder zusätzlich kann das Meßgrößenaufnehmer-Ankoppelelement der Durchflussvorrichtung eine flexible Membran aufweisen. Durch die flexible Membran ist ein Bewegungskontakt zu dem durch die Durchflussvorrichtung strömenden Blut ermöglicht.

Die mechanische Flexibilität der Membran ermöglicht beispielsweise eine schnelle und unkomplizierte Ankopplung eines als Druckmeßgrößenaufnehmer ausgebildeten Meßgrößenaufnehmers des erfindungsgemäßen Blutuntersuchungsgeräts. Mittels einer solchen Membran kann nämlich zum Beispiel eine Druckerhöhung des Bluts in einer Durchflussvorrichtung beziehungsweise in einem extrakorporalen Blutkreislauf, die eine Wölbung der Membran nach außen hervorruft, unmittelbar auf den Druckmeßgrößenaufnehmer übertragen werden.

Bei einer ganz besonders vorteilhaften und insbesondere kompakt ausbildbaren Ausführungsform sind sowohl das Meßgrößenaufnehmer-Ankoppelelement, als auch der Durchflussvorrichtungs-Meßgrößenaufnehmer (und ggf. weitere derartige Elemente) tangential um die Längsachse der Durchflussvorrichtung umlaufend und/oder relativ zueinander axial beabstandet angeordnet. Insbesondere können sie in einer gemeinsamen Ebene senkrecht zur Durchflussrichtung angeordnet sein. Hierdurch ist die Durchflussvorrichtung bezüglich ihrer Längenausdehnung in vorteilhafter Weise kurz und somit kompakt ausgebildet. So kann das Gerät auch im Bereich von Schlauchbiegungen eingesetzt werden bzw. behindert nicht eine flexible Schlauchführung. Üblicherweise werden Messaufnehmer longitudinal in Flussrichtung oder an verschiedenen Positionen angeordnet.

Bei einer weiteren Ausführungsform weist die Durchflussvorrichtung Steckanschlüsse zum fluidischen Verbinden mit Schläuchen bzw. Schlauchabschnitten eines extrakorporalen Blutkreislaufs auf. Die Schläuche können auf die Steckanschlüsse, insbesondere rutschfest und formschlüssig, aufgesteckt werden. Insbesondere ist dadurch gewährleistet, dass die Schnittstelle zwischen dem Schlauch und der Durchflussvorrichtung flüssigkeitsdicht ist, so dass kein Blut aus dem extrakorporalen Blutkreislauf tritt.

Insbesondere können die Steckanschlüsse jeweils eine Scheibe aufweisen. Die Scheiben können jeweils als ein Anschlagelement für den jeweils aufzusteckenden Schlauch fungieren. Alternativ oder zusätzlich können die Scheiben derart angeordnet sein, dass sie die Durchflussvorrichtung und ein Blutuntersuchungsgerät während eines Ankopplungsvorganges relativ zueinander mechanisch führen, wobei das Blutuntersuchungsgerät zwischen den Scheiben geführt auf den dazwischen liegenden Teil der Durchflussvorrichtung, insbesondere formschlüssig, aufgesteckt werden kann.

Bei einer besonders vorteilhaften Ausführungsform ist die Durchflussvorrichtung aus einem flexiblen Material, insbesondere einem Kunststoff, hergestellt. Dadurch wird das Risiko eines Bruchs oder einer Zerstörung der Durchflussvorrichtung bei einem mechanischen Schlag reduziert, da sie aufgrund ihrer Flexibilität bis zu einem bestimmten Grad ihre Form ohne eine Verletzung ihrer Struktur verändern kann. Insbesondere kann die Durchflussvorrichtung einen flexiblen durchsichtigen Schlauch aufweisen oder in Form eines flexiblen durchsichtigen Schlauchs samt ihren technischen Einrichtungen ausgebildet sein, wobei das Blut durch den Schlauch strömt. Der durchsichtige Schlauch ermöglicht neben dem bereits angeführten Vorteil bezüglich der Flexibilität in vorteilhafter Weise eine unmittelbare Beobachtung des Bluts. Das Blutuntersuchungsgerät kann vorteilhaft auch in der Weise ausgebildet sein, dass es mit einem einfachen, insbesondere durchsichtigen, Stück Schlauch als Durchflussvorrichtung (ohne einen integrierten Meßgrößenaufnehmer) in Wirkverbindung treten kann, um Blutparameter zu erfassen.

Die erfindungsgemäße Durchflussvorrichtung kann alternativ als eine Küvette aus Polycarbonat oder MABS ausgebildet sein.

Von besonderem Vorteil ist ein System, das die vorliegende Durchflussvorrichtung und das erfindungsgemäße Blutuntersuchungsgerät aufweist. Dadurch wird es dem medizinischen Personal ermöglicht, Blutparameter in einem extrakorporalen Blutkreislauf eines Patienten, der insbesondere an einer Herz-Lungen-Maschine angeschlossen ist, schnell und einfach zu messen und/oder zu überwachen.

In der Zeichnung ist der Erfindungsgegenstand beispielhaft und schematisch dargestellt und wird anhand der Figuren nachfolgend beschrieben, wobei gleiche oder gleich wirkende Elemente auch in unterschiedlichen Ausführungsbeispielen zumeist mit denselben Bezugszeichen versehen sind. Dabei zeigen:
- Fig. 1: ein erstes Ausführungsbeispiel eines erfindungsgemäßen Systems, das ein erstes Ausführungsbeispiel eines Blutuntersuchungsgeräts und ein erstes Ausführungsbeispiel einer Durchflussvorrichtung umfasst, in einer Draufsicht,
- Fig. 2: einen extrakorporalen Blutkreislauf mit dem erfindungsgemäßen System,
- Fig. 3: ein zweites Ausführungsbeispiel eines erfindungsgemäßen Systems, das ein zweites Ausführungsbeispiel des erfindungsgemäßen Blutuntersuchungsgeräts und ein zweites Ausführungsbeispiel der Durchflussvorrichtung umfasst, in einer Schnittdarstellung,
- Fig. 4: ein drittes Ausführungsbeispiel eines erfindungsgemäßen Systems, das ein drittes Ausführungsbeispiel des erfindungsgemäßen Blutuntersuchungsgeräts und ein drittes Ausführungsbeispiel der Durchflussvorrichtung umfasst, in einer Schnittdarstellung,
- Fig. 5: ein viertes Ausführungsbeispiel der Durchflussvorrichtung in einer perspektivischen Ansicht, und
- Fig. 6: ein fünftes Ausführungsbeispiel der Durchflussvorrichtung in einer Schnittdarstellung.

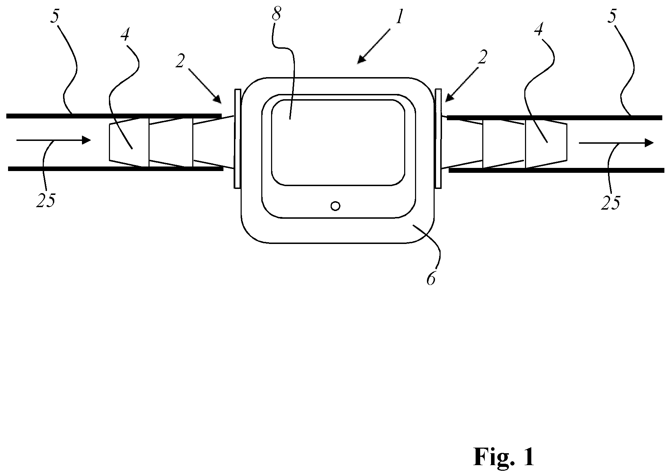

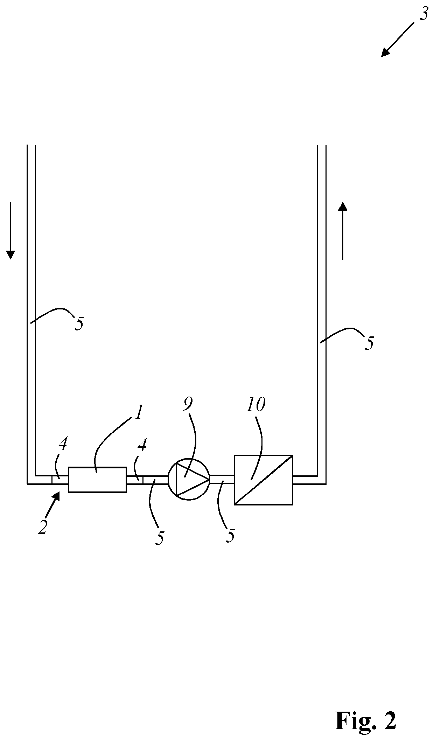

Die Figuren 1 und 2 zeigen ein Ausführungsbeispiel eines erfindungsgemäßen Systems, das ein Blutuntersuchungsgerät 1 und eine Durchflussvorrichtung 2 umfasst, in einer Draufsicht. Das Blutuntersuchungsgerät 1 ist an eine Durchflussvorrichtung 2 eines extrakorporalen Blutkreislaufs 3 angekoppelt. Das Blutuntersuchungsgerät 1 ist zum Erfassen von Blutparametern des durch die Durchflussvorrichtung 2 strömenden Blutes ausgebildet.

Die Durchflussvorrichtung 2 weist zwei Steckanschlüsse 4 auf, die mit Schläuchen 5 eines extrakorporalen Blutkreislaufs 3 verbunden sind. Die Durchflussrichtung 25 ist in den Figuren 1 und 2 durch Pfeile gekennzeichnet.

Das Blutuntersuchungsgerät 1 weist ein Gehäuse 6 auf, in bzw. an dem ein (in den Figuren 1 und 2 nicht dargestellter) elektrischer Energiespeicher 7 und eine Anzeigevorrichtung zum Anzeigen 8 der erfassten Blutparamete angeordnet sind.

Das Blutuntersuchungsgerät 1 des Systems kann schnell und ohne auf den Anschluss an ein Stromversorgungsnetz oder an andere Geräte angewiesen zu sein zum Einsatz gebracht werden. Das Blutuntersuchungsgerät 1 wird ohne Kabel betrieben, so dass einerseits eine besonders gute Handhabbarkeit erzielt ist und andererseits eine Behinderung des den Patienten behandelnden Personals durch herumhängende Kabel vermieden ist oder gar ein Abreißen der Kabel durch umhergehendes Personal verhindert ist. Außerdem ist das Blutuntersuchungsgerät 1 als Handgerät besonders kompakt ausgebildet.

Figur 2 zeigt einen extrakorporalen Blutkreislauf 3 mit der Durchflussvorrichtung 2, an die das Blutuntersuchungsgerät 1 angekoppelt ist. Der extrakorporale Blutkreislauf 3 weist außerdem eine Pumpe 9 zum Pumpen von Blut und einen Oxygenator 10 auf. Die Geräte sind mittels Schläuchen 5 verbunden, wobei die Verbindung zum Blutkreislauf des Patienten ebenfalls mittels Schläuchen 5 erfolgt.

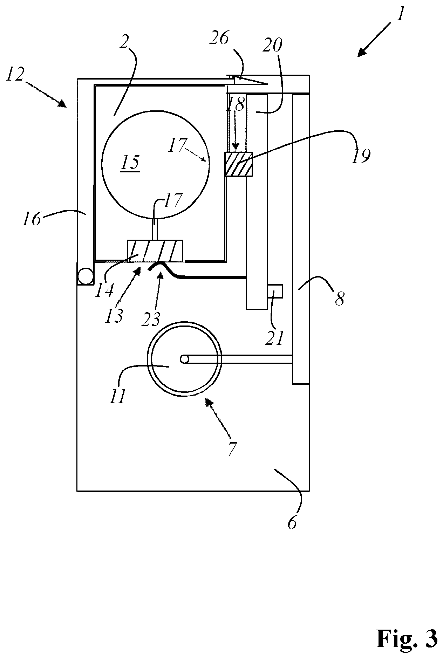

Fig. 3 zeigt ein zweites Ausführungsbeispiel eines erfindungsgemäßen Systems, das ein zweites Ausführungsbeispiel eines Blutuntersuchungsgeräts und ein zweites Ausführungsbeispiel einer Durchflussvorrichtung umfasst, in einer Schnittdarstellung. Das Blutuntersuchungsgerät 1 ist autark ausgebildet und kann ohne einen Kabelanschluss an übrige Geräte einer Herz-Lungen-Maschine oder eine externe Energiequelle betrieben werden. Das Blutuntersuchungsgerät 1 weist einen elektrischen Energiespeicher 7 auf, der bei dem gezeigten Ausführungsbeispiel eine Batterie 11 ist, wobei diese die übrigen Komponenten des Blutuntersuchungsgeräts 1 mit elektrischer Energie versorgt.

Das Blutuntersuchungsgerät 1 weist eine Aufnahme 12 auf, in der die Durchflussvorrichtung 2 festgelegt ist. Die Aufnahme 12 weist eine an dem Gehäuse 6 drehbar gelagerte Klappe 16 auf, die zum Einlegen der Durchflussvorrichtung 2 geöffnet und nach dem Einlegen der Durchflussvorrichtung 2 wieder verschlossen werden kann, so dass die Durchflussvorrichtung 2 an dem Blutuntersuchungsgerät 1 formschlüssig festgelegt ist. Der obere Teil der Klappe 16 ist elastisch und weist an seinem Ende eine Rastnase 26 auf.

Die Durchflussvorrichtung 2 ist zerstörungsfrei und werkzeugfrei an der Aufnahme 12 festlegbar. Die Aufnahme 12 ermöglicht ein schnelles und effizientes An- und Abkoppeln der Durchflussvorrichtung 2. Außerdem ist durch die Aufnahme 12 für einen Benutzer eine sofort erkennbare Festlegemöglichkeit geschaffen, wodurch das Risiko einer Fehlbedienung durch fehlerhaftes Ankoppeln reduziert ist. Insbesondere ist so gewährleistet, dass das Blutuntersuchungsgerät 1 und die Durchflussvorrichtung 2 korrekt und unversehrt miteinander gekoppelt werden.

Die Durchflussvorrichtung 2 weist ein Meßgrößenaufnehmer-Ankoppelelement 17 und das Blutuntersuchungsgeräts 1 einen Meßgrößenaufnehmer 18 auf. Der Meßgrößenaufnehmer 18 ist ein optischer Meßgrößenaufnehmer 19. Das Meßgrößenaufnehmer-Ankoppelelement 17 ist durch die transparente Wand der Durchflussvorrichtung 2 gebildet, durch die Licht zum Erfassen eines optischen Messsignals hindurchtreten kann.

Beim Einfügen der Durchflussvorrichtung 2 wird automatisch eine Wirkverbindung des Meßgrößenaufnehmers 18 des Blutuntersuchungsgerät 1 an das Meßgrößenaufnehmer-Ankoppelelement 17 der Durchflussvorrichtung 2 und automatisch eine Wirkverbindung der Meßgrößenaufnehmerschnittstelle 23 an den Durchflussvorrichtungs-Meßgrößenaufnehmer 13 der Durchflussvorrichtung 2 hergestellt. Hierdurch ergibt sich in vorteilhafter Weise eine effiziente Ankopplung der Durchflussvorrichtung 2 an das Blutuntersuchungsgerät 1. Es können vorteilhaft auch Daten von dem Meßgrößenaufnehmer 13 an das Blutuntersuchungsgerät 1 über die Meßgrößenaufnehmerschnittstelle 23 übertragen werden.

Die Durchflussvorrichtung 2 weist einen Durchflussvorrichtungs-Meßgrößenaufnehmer 13 auf, der bei dem in der Figur 3 dargestellten Ausführungsbeispiel ein Druckmeßgrößenaufnehmer 14 ist. Der Druckmeßgrößenaufnehmer 14 ist mittels eines Meßgrößenaufnehmer-Ankoppelelements 17 mit dem Innenraum 15 der Durchflussvorrichtung 2, durch den das Blut strömt, verbunden, so dass der im Innenraum auftretende Druck des Bluts zu dem Druckmeßgrößenaufnehmer 14 übertragen wird. Das Meßgrößenaufnehmer-Ankoppelelement 17 weist eine Membran auf, mittels der der Druck des Blutes auf den Druckmeßgrößenaufnehmer 14 übertragen wird.

Das Meßgrößenaufnehmer-Ankoppelelement 17 kann alternativ zum Beispiel auch durch einen Druckstempel gebildet sein, der auf den Druckmeßgrößenaufnehmer 14 wirkt.

Das Blutuntersuchungsgerät 1 weist außerdem eine elektronische Einrichtung 20, wie zum Beispiel einen Programmable logic controller (PCL), und eine Anzeigevorrichtung zum Anzeigen 8 der erfassten Blutparameter auf.

Außerdem weist das Blutuntersuchungsgerät 1 eine Übertragungs- und Empfangsvorrichtung 21 zum Senden und zum Empfangen von Messsignalen auf, wobei die Übertragungs- und Empfangsvorrichtung 21 bei diesem Ausführungsbeispiel zur drahtlosen Übertragung ausgebildet ist.

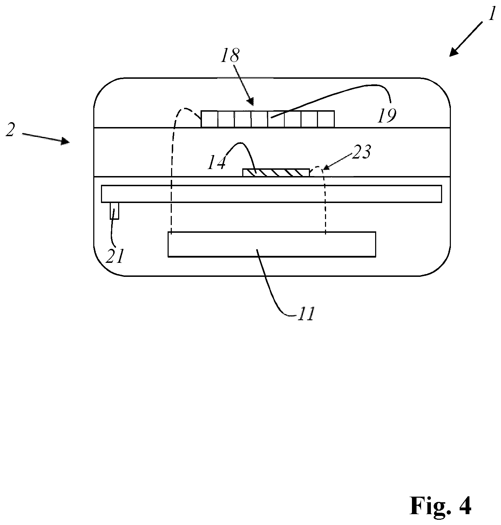

Figur 4 zeigt ein drittes Ausführungsbeispiel eines erfindungsgemäßen Blutuntersuchungsgeräts 1 in einer Schnittdarstellung. Das Blutuntersuchungsgerät 1 weist als Meßgrößenaufnehmer 18 einen optischen Meßgrößenaufnehmer 19 auf, der gegenüber dem Druckmeßgrößenaufnehmer 14 der Durchflussvorrichtung 2 angeordnet ist.

Die Batterie 11 des Blutuntersuchungsgeräts 1 versorgt sowohl den optischen Meßgrößenaufnehmer 19 des Blutuntersuchungsgeräts 1, als auch den Druckmeßgrößenaufnehmer 14 der Durchflussvorrichtung 2, mit elektrischer Energie. Die Batterie 11 ist mittels eines Kabels mit dem Meßgrößenaufnehmer 19 verbunden und mit dem Druckmeßgrößenaufnehmer 14 mittels der schematisch angedeuteten Meßgrößenaufnehmerschnittstelle 23 gekoppelt.

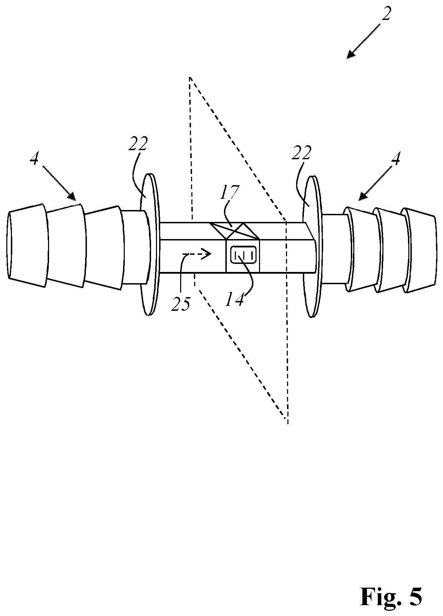

Figur 5 zeigt ein viertes Ausführungsbeispiel einer erfindungsgemäßen Durchflussvorrichtung 2 in einer perspektivischen Ansicht.

Die Durchflussvorrichtung 2 weist zwei Steckanschlüsse 4 zum fluidischen Verbinden mit (in dieser Figur nicht dargestellten) Schläuchen 5 eines (in dieser Figur nicht dargestellten) extrakorporalen Blutkreislaufs 3 auf. Die Steckanschlüsse 4 weisen jeweils eine Scheibe 22 auf. Die Scheiben 22 fungieren als Anschlag für die aufzusteckenden Schläuche 5. Die Scheiben 22 erfüllen gleichzeitig eine Doppelfunktion, nämlich die Führung eines an die Durchflussvorrichtung 2 anzukoppelnden Blutuntersuchungsgerät 1, sobald es auf die Durchflussvorrichtung 2 zur Ankopplung aufgesteckt wird. Außerdem sind die Scheiben 22 derart angeordnet, dass sie eine formschlüssige Ankopplung des Blutuntersuchungsgeräts 1 bewirken.

Die Scheiben sind also derart angeordnet, dass sie die Führung eines an der Durchflussvorrichtung 2 zu befestigenden Blutuntersuchungsgeräts 1 übernehmen, wobei das Blutuntersuchungsgerät 1 zwischen den Scheiben 5 geführt auf den dazwischen liegenden Teil der Durchflussvorrichtung 2 formschlüssig aufgesteckt wird.

Die Durchflussvorrichtung 2 weist außerdem einen Druckmeßgrößenaufnehmer 14 und ein Meßgrößenaufnehmer-Ankoppelelement 17 zum automatischen Ankoppeln eines Meßgrößenaufnehmers 18 (in dieser Figur nicht dargestellt) eines Blutuntersuchungsgeräts 1 auf. Das Meßgrößenaufnehmer-Ankoppelelement 17 und der Druckmeßgrößenaufnehmer 14 sind in einer gemeinsamen Ebene (schematisch gestrichelt dargestellt) senkrecht zur Durchflussrichtung 25 angeordnet. Hierdurch ist die Durchflussvorrichtung 2 bezüglich ihrer Längenausdehnung kurz und somit kompakt ausgebildet.

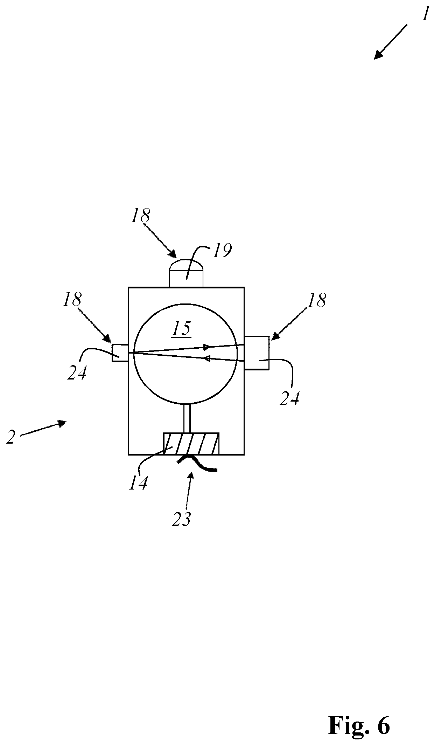

Fig. 6 zeigt ein fünftes Ausführungsbeispiel einer erfindungsgemäßen Durchflussvorrichtung 2 in einer Schnittdarstellung.

Die Durchflussvorrichtung 2 ist an einem erfindungsgemäßen Blutuntersuchungsgerät 1 festgelegt, wobei zur Vereinfachung nur die Meßgrößenaufnehmer 18 des Blutuntersuchungsgeräts 1 dargestellt sind. Das Blutuntersuchungsgerät 1 weist einen Meßgrößenaufnehmer 18 auf, der als Ultraschall-Meßgrößenaufnehmer 24 den Blutdurchfluss pro Zeit aufnimmt. Der Ultraschall-Meßgrößenaufnehmer 24 weist einen Ultraschallsender und einen Reflektor zur Reflektion der Ultraschallwellen auf. Außerdem weist das (nicht vollständig dargestellte) Blutuntersuchungsgerät 1 einen optischen Meßgrößenaufnehmer 19, zur Bestimmung des Sauerstoffgehalts und/oder CO2 Gehalts im Blut, auf.

Dadurch, dass mehrere Meßgrößenaufnehmer 18 unterschiedlicher Art vorhanden sind, können verschiedene technische Untersuchungsmöglichkeiten, auch in Kombination, realisiert werden.

### Bezugszeichenliste:

- 1: Blutuntersuchungsgerät
- 2: Durchflussvorrichtung
- 3: Blutkreislauf
- 4: Steckanschluss
- 5: Schlauch
- 6: Gehäuse
- 7: Energiespeicher
- 8: Anzeigevorrichtung zum Anzeigen
- 9: Pumpe
- 10: Oxygenator
- 11: Batterie
- 12: Aufnahme
- 13: Durchflussvorrichtungs-Meßgrößenaufnehmer
- 14: Druckmeßgrößenaufnehmer
- 15: Innenraum
- 16: Klappe
- 17: Meßgrößenaufnehmer-Ankoppelelement
- 18: Meßgrößenaufnehmer
- 19: optischer Meßgrößenaufnehmer
- 20: Programmable logic controller
- 21: Übertragungs- und Empfangsvorrichtung
- 22: Scheibe
- 23: Meßgrößenaufnehmerschnittstelle
- 24: Ultraschall-Meßgrößenaufnehmer
- 25: Durchflussrichtung
- 26: Rastnase

## Patentansprüche

1. Blutuntersuchungsgerät (1) zum Erfassen von Blutparametern von Blut in einem extrakorporalen Blutkreislauf (3) außerhalb einer Herz-Lungen-Maschine, wobei das Blutuntersuchungsgerät (1) eine Aufnahme (12) zum unmittelbaren Ankoppeln an eine Durchflussvorrichtung (2) für Blut aufweist, welche mit dem extrakorporalen Blutkreislauf (3) verbindbar ist, ***dadurch gekennzeichnet, dass*** das Blutuntersuchungsgerät (1) autark ausgebildet ist und ohne einen Kabelanschluss an übrige Geräte einer Herz-Lungen-Maschine betrieben werden kann, wobei das Blutuntersuchungsgerät (1) einen Meßgrößenaufnehmer (18) und eine Anzeigevorrichtung (8) zum Anzeigen von mittels des Meßgrößenaufnehmers (18) erfassten und an die Anzeigevorrichtung (8) weitergeleiteten Blutparametern aufweist.

2. Blutuntersuchungsgerät (1) nach Anspruch 1, **dadurch gekennzeichnet, dass** das Blutuntersuchungsgerät (1) ein Gehäuse (6) aufweist, in oder an dem die Anzeigevorrichtung (8) zum Anzeigen der erfassten Blutparameter angeordnet ist.

3. Blutuntersuchungsgerät (1) nach Anspruch 1 oder 2, **dadurch gekennzeichnet, dass** der Meßgrößenaufnehmer (18)
a. wenigstens eine Lichtquelle zum Beaufschlagen des durch eine Durchflussvorrichtung (2) fließenden Blutes mit Licht aufweist und/oder
b. eine Schallquelle zum Beaufschlagen des durch eine Durchflussvorrichtung (2) fließenden Blutes mit Schall aufweist und/oder
c. einen Druckmeßgrößenaufnehmer aufweist und/oder
d. einen Durchflussmeßgrößenaufnehmer aufweist und/oder
e. einen optischen Meßgrößenaufnehmer (19) aufweist.

4. Blutuntersuchungsgerät (1) nach einem der Ansprüche 1 bis 3, **gekennzeichnet durch** wenigstens eine Meßgrößenaufnehmerschnittstelle (23).

5. Blutuntersuchungsgerät (1) nach Anspruch 4, **dadurch gekennzeichnet, dass** die Meßgrößenaufnehmerschnittstelle (23) dazu ausgebildet ist,
a. Energie von dem elektrischen Energiespeicher (7) zu einem in eine Durchflussvorrichtung (2) integrierten Durchflussvorrichtungs-Meßgrößenaufnehmer (13) zu übertragen und/oder
b. dazu ausgebildet ist, Messsignale von einem in eine Durchflussvorrichtung (2) integrierten Durchflussvorrichtungs-Meßgrößenaufnehmer (13) weiter zu leiten.

6. Blutuntersuchungsgerät (1) nach Anspruch 4 oder 5, **dadurch gekennzeichnet, dass** die Meßgrößenaufnehmerschnittstelle (23) zur Ankopplung an einen Durchflussvorrichtungs-Meßgrößenaufnehmer (13) ausgebildet ist, der einen Druckmeßgrößenaufnehmer (14) oder einen Durchfluss-Meßgrößenaufnehmer oder einen optischen Meßgrößenaufnehmer (19) aufweist.

7. Blutuntersuchungsgerät (1) nach einem der Ansprüche 1 bis 6, **gekennzeichnet durch** eine Aufnahme (12), in oder an der eine Durchflussvorrichtung (2) festlegbar ist.

8. Blutuntersuchungsgerät (1) nach einem der Ansprüche 1 bis 7, **dadurch gekennzeichnet, dass** das Blutuntersuchungsgerät (1) dazu ausgebildet ist, wenigstens einen der folgenden Blutparameter zu erfassen:
a. Sauerstoffsättigung des Bluts,
b. CO2 Gehalt des Bluts
c. Temperatur des Bluts,
d. Druck des Bluts,
e. Durchflussmenge des Bluts pro Zeiteinheit,
f. Durchflussgeschwindigkeit des Bluts,
g. Hämoglobinanteil im Blut.

9. System aufweisend eine Durchflussvorrichtung (2), die dazu ausgebildet und bestimmt ist, in einen extrakorporalen Blutkreislauf (3) derart eingefügt zu werden, dass Blut entlang einer Durchflussrichtung (25) durch die Durchflussvorrichtung (2) strömt und die zur unmittelbaren Ankopplung an ein Blutuntersuchungsgerät (1) ausgebildet ist, wobei die Durchflussvorrichtung (2) wenigstens ein Meßgrößenaufnehmer-Ankoppelelement (17) und wenigstens einen Durchflussvorrichtungs-Meßgrößenaufnehmer (13) umfasst, und aufweisend ein Blutuntersuchungsgerät (1) nach einem der vorhergehenden Ansprüche.

10. System nach Anspruch 9, **dadurch gekennzeichnet, dass**
a. der Durchflussvorrichtungs-Meßgrößenaufnehmer (13) einen Druckmeßgrößenaufnehmer (14) oder einen Durchflussmeßgrößenaufnehmer oder einen optischen Meßgrößenaufnehmer aufweist, und/oder dass
b. der Durchflussvorrichtungs-Meßgrößenaufnehmer (13) wenigstens eine Lichtquelle und/oder eine Schallquelle zum Beaufschlagen des durch die Durchflussvorrichtung (2) fließenden Blutes mit Licht und/oder Schall aufweist und/oder dass
c. das Meßgrößenaufnehmer-Ankoppelelement (17) zur Ankopplung an einen Meßgrößenaufnehmer (18) eines Blutuntersuchungsgeräts (1), der einen Druckmeßgrößenaufnehmer (14) oder einen Durchflussmeßgrößenaufnehmer oder einen optischen Meßgrößenaufnehmer aufweist, ausgebildet ist und/oder dass
d. das Meßgrößenaufnehmer-Ankoppelelement (17) ein Fenster oder eine flexible Membran aufweist.

11. System nach Anspruch 9 oder 10, **dadurch gekennzeichnet, dass** sowohl das Meßgrößenaufnehmer-Ankoppelelement (17) als auch der Durchflussvorrichtungs-Meßgrößenaufnehmer (13) tangential um die Längsachse der Durchflussvorrichtung (2) umlaufend und/oder relativ zueinander axial beabstandet angeordnet sind.

## Claims

1. Blood testing device (1) for detecting blood parameters of blood in an extracorporeal blood circuit (3) outside a heart-lung machine, wherein the blood testing device (1) has a holder (12) for direct coupling to a throughflow apparatus (2), which can be connected to the extracorporeal blood circuit (3), ***characterised in that*** the blood testing device (1) is designed to be autonomous and can be operated without a cable connection to other devices of a heart-lung machine,
wherein the blood testing device (1) has a transducer (18) and a display apparatus (8) for displaying blood parameters detected by means of the transducer (18) and forwarded to the display apparatus (8).

2. Blood testing device (1) according to claim 1, **characterised in that** the blood testing device (1) has a housing (6) in or on which the display apparatus (8) is arranged for displaying the detected blood parameters.

3. Blood testing device (1) according to claim 1 or 2, **characterised in that** the transducer (18)
a. has at least one light source for applying light to the throughflow apparatus (2) and/or
b. has a sound source for applying sound to the throughflow apparatus (2) and/or
c. has a pressure transducer and/or
d. has a flow rate transducer and/or
e. has an optical transducer (19).

4. Blood testing device (1) according to one of claims 1 to 3, **characterised by** at least one transducer interface (23).

5. Blood testing device (1) according to claim 4, **characterised in that** the transducer interface (23) is designed to
a. transfer energy from the electrical energy store (7) to a throughflow apparatus transducer (13) integrated into a throughflow apparatus (2) and/or
b. forward measurement signals from a throughflow apparatus transducer (13) integrated into a throughflow apparatus (2).

6. Blood testing device (1) according to claim 4 or 5, **characterised in that** the transducer interface (23) is designed for coupling to a throughflow apparatus transducer (13) which has a pressure transducer (14) or a flow rate transducer or an optical transducer (19).

7. Blood testing device (1) according to one of claims 1 to 6, **characterised by** a receptacle (12), in or on which a throughflow apparatus (2) can be fixed.

8. Blood testing device (1) according to one of claims 1 to 7, **characterised in that** the blood testing device (1) is designed to detect at least one of the following blood parameters:
a. Oxygen saturation of the blood,
b. CO₂ content of the blood,
c. Temperature of the blood,
d. Pressure of the blood,
e. Flow quantity of the blood per unit of time,
f. Flow speed of the blood,
g. Haemoglobin level in the blood.

9. System having a throughflow apparatus (2), which is designed and intended to be inserted into an extracorporeal blood circuit (3) in such a way that blood flows through the throughflow apparatus (2) along a flow direction (25) and which is designed for direct coupling to a blood testing device (1), wherein the throughflow apparatus (2) comprises at least one transducer coupling element (17) and at least one throughflow apparatus transducer (13), and having a blood testing device (1) according to one of the preceding claims.

10. System according to claim 9, **characterised in that**
a. the throughflow apparatus transducer (13) has a pressure transducer (14) or a flow rate transducer or an optical transducer and/or
b. the throughflow apparatus transducer (13) has at least one light source and/or one sound source for applying light and/or sound to the throughflow apparatus (2) and/or
c. the transducer coupling element (17) is designed for coupling to a transducer (18) of a blood testing device (1) which has a pressure transducer (14) or a flow rate transducer or an optical transducer and/or
d. the transducer coupling element (17 has a window or a flexible membrane.

11. System according to claim 9 or 10, **characterised in that** both the transducer coupling element (17) and the throughflow apparatus transducer (13) are arranged tangentially around the longitudinal axis of the throughflow apparatus (2) and/or at an axial distance relative to one another.

## Revendications

1. Appareil d'analyse du sang (1) permettant de détecter les paramètres sanguins du sang dans un circuit sanguin extracorporel (3) à l'extérieur d'une machine cœur-poumon, l'appareil d'analyse du sang (1) comportant un logement (12) destiné à être couplé directement à un dispositif d'écoulement (2) pour le sang, qui peut être relié à la circulation sanguine extracorporelle (3), ***caractérisé en ce que*** l'appareil d'analyse du sang (1) est conçu de manière autonome et peut être utilisé sans connexion par câble à d'autres appareils d'une machine cœur-poumon,
l'appareil d'analyse du sang (1) comportant un capteur de capteur de mesure (18) et un dispositif d'affichage (8) permettant d'afficher des paramètres sanguins détectés au moyen du capteur de mesure (18) et transmis au dispositif d'affichage (8).

2. Appareil d'analyse du sang (1) selon la revendication 1, **caractérisé en ce que** l'appareil d'analyse du sang (1) comprend un boîtier (6) dans ou sur lequel est disposé le dispositif d'affichage (8) destiné à afficher les paramètres sanguins détectés.

3. Appareil d'analyse du sang (1) selon l'une des revendications 1 ou 2, **caractérisé en ce que** le capteur de mesure (18)
a. comporte au moins une source lumineuse permettant d'exposer à la lumière le sang s'écoulant à travers un dispositif d'écoulement (2) et/ou
b. comporte une source sonore permettant d'exposer à des ondes sonores le sang s'écoulant à travers un dispositif d'écoulement (2) et/ou
c. comporte un capteur de mesure de pression et/ou
d. comporte un capteur de mesure de débit et/ou
e. comporte un capteur de mesure optique (19).

4. Appareil d'analyse du sang (1) selon l'une des revendications 1 à 3, **caractérisé par** au moins une interface du capteur de mesure (23).

5. Appareil d'analyse du sang (1) selon la revendication 4, **caractérisé en ce que** l'interface du capteur de mesure (23) est conçue pour
a. transférer l'énergie du dispositif de stockage d'énergie électrique (7) vers un capteur de mesure du dispositif d'écoulement (13) intégré dans un dispositif d'écoulement (2) et/ou
b. est conçue pour transmettre les signaux de mesure provenant d'un capteur de mesure du dispositif d'écoulement (13) intégré dans un dispositif d'écoulement (2).

6. Appareil d'analyse du sang (1) selon l'une des revendications 4 ou 5, **caractérisé en ce que** l'interface du capteur de mesure (23) est destinée à être couplée à un capteur de mesure à dispositif d'écoulement (13) qui comprend un capteur de mesure de pression (14) ou un capteur de mesure de débit ou un capteur de mesure optique (19).

7. Appareil d'analyse du sang (1) selon l'une des revendications 1 à 6, **caractérisé par** un logement (12), dans ou sur lequel un dispositif d'écoulement (2) peut être fixé.

8. Appareil d'analyse du sang (1) selon l'une des revendications 1 à 7, **caractérisé en ce que** l'appareil d'analyse du sang (1) est conçu pour mesurer au moins l'un des paramètres sanguins suivants :
a. Saturation du sang en oxygène,
b. Teneur du sang en CO2
c. Température du sang,
d. Pression du sang,
e. Débit sanguin par unité de temps,
f. Vitesse d'écoulement du sang,
g. Taux d'hémoglobine dans le sang.

9. Système comprenant un dispositif d'écoulement (2) qui est conçu et destiné à être inséré dans un circuit sanguin extracorporel (3) de telle sorte que le sang s'écoule à travers le dispositif d'écoulement (2) dans le sens de l'écoulement (25) et qui est conçu pour être directement raccordé à un appareil d'analyse sanguine (1), le dispositif d'écoulement (2) comprenant au moins un élément de couplage de capteur de mesure (17) et au moins un capteur de mesure de dispositif d'écoulement (13), et comprenant un appareil d'analyse du sang (1) selon l'une des revendications précédentes.

10. Système selon la revendication 9, **caractérisé en ce que**
a. le capteur de mesure de dispositif d'écoulement (13) comporte un capteur de mesure de pression (14) ou un capteur de mesure de débit ou un capteur de mesure optique, et/ou **en ce que**
b. le capteur de mesure du dispositif d'écoulement (13) comporte au moins une source lumineuse et/ou une source sonore permettant d'exposer la sang s'écoulant à travers le dispositif d'écoulement (2) à la lumière et au son et/ou **en ce que**
c. l'élément de couplage du capteur de mesure (17) est conçu pour être couplé à un capteur de mesure (18) d'un appareil d'analyse du sang (1) qui comporte un capteur de mesure de pression (14) ou un capteur de mesure de débit ou un capteur de mesure optique, et/ou **en ce que**
d. l'élément de couplage du capteur de mesure (17 comporte une fenêtre ou une membrane flexible.

11. Système selon la revendication 9 ou 10, **caractérisé en ce que** l'élément de couplage du capteur de mesure (17) et le capteur de mesure du dispositif d'écoulement (13) sont disposés tangentiellement autour de l'axe longitudinal du dispositif d'écoulement (2) et/ou à distance axiale l'un par rapport à l'autre.
